# FASCICULE DE BREVET EUROPEEN

(11) **EP 0 845 282 B1**
(45) Date de publication et mention de la délivrance du brevet: **29.09.2004**
(21) Numéro de dépôt: 97402799.7
(22) Date de dépôt: 21.11.1997
(51) Int. Cl.: A61N 1/368, A61N 1/362

(54) **Dispositif médical implantable actif, notamment stimulateur cardiaque, défibrillateur et/ou cardioverteur, pourvu d'une fonction de stimulation double chambre et d'un mode de repli**
Implantierbare medizinisch-aktive Vorrichtung, insbesondere Herzschrittmacher, Defibrillator, und/oder Kardioverter, mit einer Zweikammerfunktion und einem alternativem Betriebsmodus
Active medical implantable device, in particular pacemaker, defibrillator and/or cardioverter equipped with a double-chamber stimulation function and an alternative mode

(30) Priorité: 22.11.1996 FR 9614270
(43) Date de publication de la demande: 03.06.1998
(73) Titulaire: ELA MEDICAL (Société anonyme), 92541 Montrouge (FR)
(72) Inventeur: Bouhour, Anne, 92410 Ville D'Avray (FR); Limousin, Marcel, 92120 Montrouge (FR)
(74) Mandataire: Dupuis-Latour, Dominique

(56) Documents cités:
- EP-A- 0 488 840
- EP-A- 0 661 076
- EP-A- 0 676 216
- EP-A- 0 676 217
- EP-A- 0 726 082
- EP-A- 0 755 696
- WO-A-95/32758

## Description

L'invention concerne les "dispositifs médicaux implantables actifs" tels que définis par la Directive 90/385/CEE du 20 juin 1990 du Conseil des communautés européennes, plus précisément les dispositifs stimulateurs cardiaques, défibrillateurs et/ou cardioverteurs pourvus d'un fonction de stimulation double chambre pour le traitement des troubles du rythme auriculaire.

Elle vise spécifiquement le fonctionnement en mode dit "de repli" (*fallback*) et la resynchronisation à l'issue d'une phase de repli.

Ces aspects ont notamment été abordés par le FR-A-2 544 989 ainsi que le EP-A-0 488 840, tous deux au nom de ELA Médical, qui décrivent un mode de traitement des ESA et un fonctionnement en repli, correspondant en particulier à ce qui est mis en oeuvre dans le stimulateur *Chorus II 6234* de cette même société.

Le myocarde peut être sujet à des TdRA (Troubles du Rythme Auriculaire), terme générique qui recouvre diverses arythmies auriculaires telles que tachycardie, fibrillation, *flutter*, etc., troubles tous caractérisés, à la détection, par un rythme auriculaire rapide.

En l'absence de TdRA, le stimulateur opère en mode DDD, c'est-à-dire avec oreillette et ventricule associés.

Lorsque l'on détecte un TdRA, c'est-à-dire, essentiellement, lorsque le rythme auriculaire dépasse un niveau admissible, le stimulateur bascule en un mode dit "de désynchronisation" (ou "de dissociation auriculo-ventriculaire") pour stimuler le ventricule indépendamment du rythme auriculaire détecté, puisque ce rythme excessif est considéré comme pathologique.

Lorsque le rythme auriculaire revient à un niveau admissible, le stimulateur opère une "resynchronisation" (ou "réassociation auriculo-ventriculaire") pour revenir de manière progressive à un fonctionnement en DDD. Il est nécessaire que cette resynchronisation soit progressive pour détecter éventuellement une association en mode 2:1, au quel cas il ne faudrait bien entendu pas procéder à la réassociation.

L'invention concerne plus particulièrement la commande de l'étape de resynchronisation de la phase de repli.

Dans les dispositifs connus, tels que ceux décrits dans les brevets précités, on resynchronise lorsque l'on ne détecte plus de TdRA.

Mais il arrive fréquemment qu'une FA (Fibrillation Auriculaire), à l'origine du TdRA, dégénère au cours du temps et, bien qu'elle soit toujours présente, ne soit en fait plus détectée par le stimulateur. Dans cette hypothèse, celui-ci, après être correctement passé en mode de repli (désynchronisation) au début de l'arythmie, interprète - à tort - l'absence de détection du trouble comme une disparition de ce trouble, et se resynchronise alors en DDD de façon indue.

En d'autres termes, des caractéristiques de signal particulières à une FA, à savoir amplitude plus faible, rythme très irrégulier, etc. peuvent conduire à une perte de détection du signal qui est interprétée et gérée à tort par le stimulateur comme une disparition du trouble correspondant.

Le but de l'invention est de remédier à cette difficulté, en proposant de ne commander la resynchronisation, de manière conditionnelle, qu'en présence d'un rythme sinusal effectivement détecté, c'est-à-dire de ne resynchroniser que si l'on détecte réellement un rythme sinusal (vrai positif), et non pas uniquement en l'absence de détection du trouble du rythme (avec risque de faux négatif).

Plus précisément, le dispositif de l'invention est un dispositif médical implantable actif, notamment un stimulateur cardiaque, défibrillateur et/ou cardioverteur, pourvu d'un fonction de stimulation double chambre et comportant : des moyens d'analyse des rythmes auriculaire et ventriculaire ; des moyens de stimulation auriculaire et ventriculaire ; des moyens de désynchronisation de la stimulation ventriculaire lorsque le rythme auriculaire dépasse un niveau admissible ; des moyens de resynchronisation progressive en cas de retour du rythme auriculaire au niveau admissible. Les moyens de resynchronisation sont commandés conditionnellement de manière à n'être déclenchés et maintenus en fonction que si : (a) les moyens d'analyse ne détectent aucun trouble du rythme auriculaire.

Selon l'invention, les moyens de resynchronisation ne sont déclenchés et maintenus en fonction que si, outre la condition (a) : (b) les moyens d'analyse détectent la présence d'un rythme sinusal effectif.

Selon des formes de mise en oeuvre avantageuses :
- les moyens d'analyse considèrent qu'il y a présence d'un rythme sinusal effectif s'ils détectent une dépolarisation auriculaire pendant au moins une fraction prédéterminée, notamment une fraction majoritaire, d'un nombre donné de cycles successifs ; cette fraction peut d'ailleurs être paramétrable ;
- les moyens de resynchronisation ne sont déclenchés et maintenus en fonction que si, outre les conditions (a) et (b) : (c) les moyens d'analyse déterminent que la durée subsistant, lors des cycles successifs au cours de la phase de resynchronisation, entre la fin de la période réfractaire post-auriculaire et la fin de l'intervalle d'échappement auriculaire est au moins égale à une durée prédéterminée, typiquement comprise entre 450 à 500 ms.

On va maintenant décrire un exemple de mise en oeuvre de l'invention, en référence aux dessins annexés.

La figure 1 est un chronogramme montrant les différents intervalles et fenêtres définis au cours d'un cycle cardiaque.

La figure 2 est un organigramme de mise en oeuvre de la fonction de repli selon l'invention.

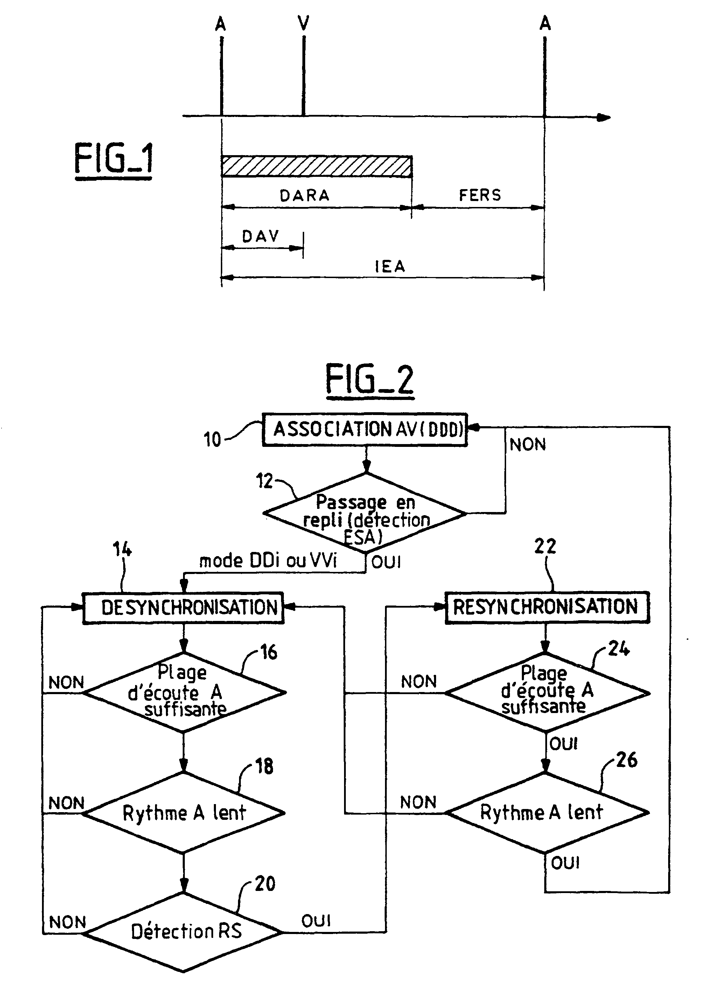

On va expliquer la fonction de repli, et notamment sa mise en oeuvre selon l'invention, en référence aux figures 1 et 2.

Initialement, le stimulateur fonctionne en mode DDD, c'est-à-dire avec association de l'oreillette et du ventricule (étape 10).

Le repli est basé sur la capacité à différencier une activité sinusale normale d'une Extra-Systole Auriculaire (ESA), qui déclenchera le passage en mode de repli. Ce type de détection (étape 12) est en lui-même connu et il ne sera pas décrit en détail. On pourra se référer notamment aux FR-A-2 544 989 et EP-A-0 488 840 précités.

Essentiellement, on définit une fenêtre de Détection de l'Accélération du Rythme Auriculaire (DARA sur la figure 1), parfois dénommée "Période Réfractaire Auriculaire Post-Auriculaire" (PRAPA). La fenêtre de DARA est une période qui démarre sur un événement auriculaire et dure une fraction donnée de l'intervalle sinusal moyen précédent. Les durées DAV et IEA correspondent, respectivement, au Délai Atrio-Ventriculaire et à l'Intervalle d'Échappement Auriculaire.

Une activité sinusale normale est définie lorsqu'un événement auriculaire est présent en dehors de la fenêtre de DARA (comme dans le cas illustré figure 1).

Inversement, on considère qu'il y a possibilité de Trouble du Rythme Auriculaire (suspicion de TdRA), et donc d'ESA, si l'on détecte un événement auriculaire à l'intérieur de la fenêtre de DARA. Si ce TdRA est confirmé sur une succession de cycles cardiaques, le stimulateur passe en mode dit "pseudo-DDI" (mode DDI ou VVI selon le rythme ventriculaire détecté), c'est-à-dire que l'on désynchronise la simulation du ventricule d'avec le rythme auriculaire détecté. La désynchronisation correspondante (étape 14) consiste à allonger progressivement la durée de l'intervalle d'échappement ventriculaire jusqu'à ce que la fréquence de stimulation atteigne une fréquence plus faible prédéterminée, par exemple la fréquence de base ou une fréquence prescrite par un capteur d'asservissement.

On procède alors à une succession de tests (étapes 16, 18 et 20) pour déterminer s'il y a lieu de maintenir ce fonctionnement désynchronisé ou, au contraire, procéder à une resynchronisation progressive entre oreillette et ventricule.

Tout d'abord (étape 16), on s'assure que l'écoute auriculaire est suffisante, c'est-à-dire que l'on dispose d'une Fenêtre d'Écoute du Rythme Sinusal (FERS sur la figure 1) de durée suffisante. La FERS est définie comme l'intervalle compris entre la fin de la période réfractaire post-auriculaire (DARA) et la survenance d'une nouvelle dépolarisation auriculaire détectée. On considère qu'il y a plage d'écoute auriculaire suffisante si, par exemple, la durée de la période FERS est d'au moins 470 ms (cette valeur n'étant bien entendu pas limitative ; typiquement, elle peut prendre une valeur de l'ordre de 450 à 500 ms).

La condition que l'on vérifie ensuite (étape 18) est la condition classique d'absence de trouble du rythme, qui serait révélé par un rythme auriculaire rapide. On sait en effet que les TdRA, qui peuvent recouvrir diverses arythmies auriculaires telles que tachycardie, fibrillation, *flutter*, etc., sont toutes caractérisés, à la détection, par un rythme auriculaire rapide. On s'assure ainsi que l'on ne détecte aucun intervalle PP court sur douze cycles consécutifs. Le seuil de détection est typiquement fixé à 500 ms, valeur en-deçà de laquelle on considère que le rythme est "lent", et "rapide", donc pathologique, au-delà.

Si l'on trouve au moins un intervalle PP court sur douze intervalles consécutifs, on considère que la condition de resynchronisation n'est pas vérifiée et l'on reste en mode désynchronisé (retour à l'étape 14).

Si, en revanche, on est en présence d'un rythme auriculaire lent, la condition correspondante pour le passage à la resynchronisation est alors vérifiée.

La troisième condition (étape 20), qui se cumule avec les deux précédentes, consiste à détecter que l'on est bien en présence d'un rythme sinusal, positivement détecté.

Le critère correspondant est, par exemple, que l'on ait moins de x % de stimulations auriculaires détectées sur un nombre prédéterminé de cycles consécutifs ; x peut-être un seuil paramétrable et/ou prendre une valeur par exemple de 50 %, c'est-à-dire que l'on doit avoir moins de six stimulations auriculaires sur les douze derniers cycles examinés (ou, en d'autres termes, au moins 50 % d'événements auriculaires détectées avec intervalle PP long).

Si les trois conditions des étapes 16 (plage d'écoute auriculaire suffisante), 18 (rythme auriculaire lent) et 20 (détection d'un rythme sinusal) sont cumulativement vérifiées, on considère que l'arythmie auriculaire est terminée et l'on passe alors au mode de resynchronisation progressive (étape 22) afin d'accélérer peu à peu le rythme ventriculaire jusqu'à atteindre le rythme auriculaire avec lequel on pourra enfin retrouver le synchronisme. La gestion de l'IEA (Intervalle d'Échappement Auriculaire) à cette fin est décrite par exemple dans les deux documents précités, auxquels on pourra se reporter, et elle est en elle-même classique.

Cette phase de resynchronisation (étape 22) n'est cependant poursuivie jusqu'à son terme que si, d'une part, la plage d'écoute auriculaire est toujours suffisante (étape 24, semblable à l'étape 16 pour sa mise en oeuvre) et s'il n'y a toujours aucun PP court sur les douze cycles (étape 26, semblable à l'étape 18 pour sa mise en oeuvre).

Si ces deux conditions sont vérifiées cumulativement, on peut se réassocier en DDD (retour à l'étape 10).

En revanche, si l'écoute auriculaire est insuffisante ou si l'on redétecte un ou plusieurs intervalles PP courts, on retourne au repli en mode DDI ou VVI (étape 14), jusqu'à ce que les trois conditions de réassociation (étapes 16, 18 et 20) indiquées ci-dessus soient à nouveau vérifiées.

## Revendications

1. Un dispositif médical implantable actif, notamment un stimulateur cardiaque, défibrillateur et/ou cardioverteur, pourvu d'un fonction de stimulation double chambre, ce dispositif comportant :
- des moyens d'analyse des rythmes auriculaire et ventriculaire,
- des moyens de stimulation auriculaire et ventriculaire,
- des moyens (14) de désynchronisation de la stimulation ventriculaire lorsque le rythme auriculaire dépasse un niveau admissible,
- des moyens (22) de resynchronisation progressive en cas de retour du rythme auriculaire au niveau admissible,
dispositif dans lequel les moyens de resynchronisation sont commandés conditionnellement de manière à n'être déclenchés et maintenus en fonction que si :
a) les moyens d'analyse ne détectent (18, 26) aucun trouble du rythme auriculaire,
dispositif **caractérisé en ce que** les moyens de resynchronisation ne sont déclenchés et maintenus en fonction que si, outre la condition (a) :
b) les moyens d'analyse détectent (20) la présence d'un rythme sinusal effectif.

2. Le dispositif de la revendication 1, dans lequel les moyens d'analyse considèrent qu'il y a présence d'un rythme sinusal effectif s'ils détectent une dépolarisation auriculaire pendant au moins une fraction prédéterminée d'un nombre donné de cycles successifs.

3. Le dispositif de la revendication 2, dans lequel ladite fraction prédéterminée est une fraction majoritaire.

4. Le dispositif de la revendication 2, dans lequel ladite fraction prédéterminée est une fraction paramétrable.

5. Le dispositif de la revendication 1, dans lequel les moyens de resynchronisation ne sont déclenchés et maintenus en fonction que si, outre les conditions (a) et (b) :
c) les moyens d'analyse déterminent (16, 24) que la durée (FERS) subsistant, lors des cycles successifs au cours de la phase de resynchronisation, entre la fin de la période réfractaire post-auriculaire (DARA) et la fin de l'intervalle d'échappement auriculaire (IEA) est au moins égale à une durée prédéterminée.

6. Le dispositif de la revendication 5, dans lequel ladite durée prédéterminée est comprise entre 450 à 500 ms.

## Patentansprüche

1. Aktive implantierbare medizinische Vorrichtung, insbesondere einen Herzschrittmacher, Defibrillator und/oder Kardioverter, ausgestattet mit einer Doppelkammerstimulationsfunktion, wobei die Vorrichtung umfasst:
- Mittel zur Analyse des atrialen und ventrikulären Rhythmus,
- Mittel zur atrialen und ventrikulären Stimulation,
- Mittel (14) zur Desynchronisation der ventrikulären Stimulation, wenn der atriale Rhythmus ein zulässiges Niveau überschreitet,
- Mittel (22) zur fortschreitenden Resynchronisation im Fall der Rückkehr des atrialen Rhythmus zum zulässigen Niveau,
Vorrichtung, in welcher die Mittel zur Resynchronisation bedingt gesteuert werden, um nur ausgelöst und in Betrieb gehalten zu werden, wenn:
a) die Mittel zur Analyse keine Störung des atrialen Rhythmus detektieren (18, 26),
wobei die Vorrichtung gekennzeichnet ist, dass die Mittel zur Resynchronisation nur ausgelöst und in Betrieb gehalten werden, wenn, außer der Bedingung (a):
b) die Mittel zur Analyse das Vorhandensein eines effektiven sinusalen Rhythmus detektieren (20).

2. Vorrichtung gemäß Anspruch 1, in welcher die Mittel zur Analyse betrachten, ob ein effektiver sinusaler Rhythmus vorhanden ist, wenn sie eine atriale Depolarisation detektieren, während mindestens eines vorherbestimmten Bruchteils einer gegebenen Anzahl von aufeinander folgenden Zyklen.

3. Vorrichtung gemäß Anspruch 2, in welchem der vorherbestimmte Bruchteil ein überwiegender Bruchteil ist.

4. Vorrichtung gemäß Anspruch 2, in welcher der vorherbestimmte Bruchteil ein parametrierbarer Bruchteil ist.

5. Vorrichtung gemäß Anspruch 1, in welcher die Mittel zur Resynchronisation nur ausgelöst und in Betrieb gehalten werden, wenn, außer den Bedingungen (a) und (b):
c) die Mittel zur Analyse bestimmen (16, 24), dass die bestehende Dauer (FERS), bei aufeinander folgenden Zyklen im Verlauf der Resynchronisationsphase zwischen dem Ende der post-atrialen Refraktärperiode (DARA) und dem Ende des atrialen Auslöseintervalls (IEA) mindestens gleich einer vorherbestimmten Dauer ist.

6. Vorrichtung gemäß Anspruch 5, in welcher die vorherbestimmte Dauer zwischen 450 und 500 ms liegt.

## Claims

1. An active implantable medical device, particularly a cardiac pacemaker, defibrillator and/or cardioverter, including a double-chamber stimulation function, said device comprising:
- means for analyzing atrial and ventricular rhythms,
- means for atrial and ventricular stimulation,
- means (14) for desynchronisation of the ventricular stimulation when the atrial rhythm exceeds an admissible level,
- means (22) for progressive resynchronisation in case the atrial rhythm returns to the admissible level,
in which device the resynchronisation means is conditionally controlled to be triggered and maintained in operation only if:
a) the analyzing means does not detect (18, 26) any trouble of the atrial rhythm,
said device being **characterised in that** the resynchronisation means is triggered and maintained in operation only if, in addition to condition (a):
b) the analyzing means detects (20) the presence of an effective sinusal rhythm.

2. The device of claim 1, wherein the analyzing means considers that there is an effective sinusal rhythm present if it detects an atrial depolarisation during at least a predetermined fraction of a given number of successive cycles.

3. The device of claim 2, wherein said predetermined fraction is a majority fraction.

4. The device of claim 2, wherein said predetermined fraction is a programmable fraction.

5. The device of claim 1, wherein the resynchronisation means is triggered and maintained in operation only if, in addition to conditions (a) and (b): c) the analyzing means determines (16, 24) that the duration (FERS) remaining, during successive cycles in the course of the resynchronisation phase, between the end of the post-atrial refractory period (DARA) and the end of the atrial escape interval (IEA) is at least equal to a predetermined duration.

6. The device of claim 5, wherein said predetermined duration is between 450 and 500 ms.
